# EUROPÄISCHE PATENTSCHRIFT

(11) **EP 3 166 659 B1**
(45) Veröffentlichungstag und Bekanntmachung des Hinweises auf die Patenterteilung: **02.09.2020**
(21) Anmeldenummer: 15736198.1
(22) Anmeldetag: 09.07.2015
(51) Int. Cl.: A61M 1/34, A61M 1/36

(54) **DISPOSABLE UND VORRICHTUNG FÜR DIE EXTRAKORPORALE BLUTBEHANDLUNG SOWIE VERFAHREN ZUR ÜBERPRÜFUNG EINER KORREKTEN LEITUNGSANKOPPLUNG**
DISPOSABLE AND DEVICE FOR EXTRACORPOREAL BLOOD TREATMENT, AND METHOD FOR CHECKING A CORRECT LINE ATTACHMENT
ARTICLE À USAGE UNIQUE ET DISPOSITIF SERVANT AU TRAITEMENT EXTRACORPOREL DU SANG, ET PROCÉDÉ PERMETTANT DE VÉRIFIER LE BON RACCORDEMENT DES LIGNES

(30) Priorität: 11.07.2014 DE 102014010306
(43) Veröffentlichungstag der Anmeldung: 17.05.2017
(73) Patentinhaber: Fresenius Medical Care Deutschland GmbH, 61352 Bad Homburg (DE)
(72) Erfinder: PUSINELLI, Thomas, 63674 Altenstadt (DE); KLEWINGHAUS, Jürgen, 61440 Oberursel (DE); VERCH, Georg, 65191 Wiesbaden (DE); WALDE, Jan-Willem, 35440 Linden (DE)
(74) Vertreter: Herrmann, Uwe
(86) Internationale Anmeldenummer: PCT/EP2015/001401
(87) Internationale Veröffentlichungsnummer: WO 2016/005056

(56) Entgegenhaltungen:
- EP-A2- 0 111 696
- DE-A1-102007 024 463
- FR-A2- 2 492 269
- US-A- 5 614 106

## Beschreibung

Die Erfindung betrifft ein Disposable und eine Vorrichtung für die extrakorporale Blutbehandlung. Die Offenbarung betrifft ferner ein Verfahren zur Überprüfung der korrekten Ankopplung einer Citratleitung an einen Flüssigkeitskreislauf einer Vorrichtung für die extrakorporale Blutbehandlung.

Die Erfindung befasst sich generell mit der Zugabe von Citrat während einer extrakorporalen Blutbehandlung, vorzugsweise während eines Aphereseverfahrens. Citrat wird typischerweise als Natriumcitrat in Lösung bzw. als Konzentrat zugegeben, sodass der Begriff Citrat im Folgenden synonym für eine citrathaltige und vorzugsweise wässrige Lösung bzw. für ein citrathaltiges und vorzugsweise wässriges Konzentrat zu verstehen ist.

Heparin und Citrat sind die beiden für extrakorporale Blutkreisläufe am häufigsten eingesetzten Substanzen mit antikoagulativer Wirkung. Die Wirkungsweise dieser beiden Substanzen unterscheidet sich grundlegend. Während Heparin einen systemischen Effekt bewirkt, d.h. über einen längeren Zeitraum im gesamten Körper antikoagulativ wirkt, ist Citrat nur lokal wirksam, d.h. nur im extrakorporalen Blutkreislauf über einen kurzen Zeitraum. Aufgrund dieses Unterschiedes ist es für die Wirkung von Heparin weitgehend irrelevant, an welcher Stelle des extrakorporealen Blutkreislaufs es injiziert wird, während Citrat möglichst nahe am Zugangsport injiziert werden sollte.

Die Wirkungsweise des Citrats umfasst die Bildung eines Chelatkomplexes mit Ca²⁺-Ionen, was eine Abreicherung von freien Ca²⁺-Ionen bewirkt. Die Ca²⁺-Ionen werden in der Leber des Patienten nach Metabolisierung des Chelatkomplexes im Rahmen des Citratzyklus wieder freigesetzt. Ca²⁺-Ionen spielen in vielen physiologischen Prozessen eine Rolle, beispielsweise in der Aktivierung des Komplementsystems, einem Bestandteil des Immunsystems. Die Abreicherung von Ca²⁺-Ionen stoppt daher die Aktivierung des Komplementsystems und hat neben einer antikoagulativen Wirkung auch eine immunsuppressive Wirkung. Eine immunsuppressive Wirkung im Blut kann in der extrakorporalen Blutbehandlung beispielsweise deshalb erwünscht sein, da Blut mit körperfremden Materialien in Verbindung tritt, die eine Immunreaktione hervorrufen könnten.

Während Citrat als antikoagulatives Mittel in der therapeutischen Apherese bereits umfassend zum Einsatz kommt, wurden auch Therapien entwickelt, in denen die immunsuppressive Wirkung genützt wird. Beispielsweise besteht eine Anwendung darin, potentielle anaphylaktische Reaktionen zu unterbinden, die durch die Kontaktierung von Blut oder Blutbestandteilen mit der Oberfläche einer Behandlungseinheit hervorgerufen werden könnten. Zu diesem Zweck wird Citrat vorzugsweise unmittelbar stromaufwärts einer Behandlungseinheit in das Plasma injiziert, da die immunsuppressive Wirkung eine bestimmte Konzentration an Citrat erfordert, was gemessen am Gesamtvolumen des Vollbluts ein Überschreiten der Toleranzgrenze für eine maximal verträgliche Verabreichung zur Folge haben kann. Dies könnte eine systemische Wirkung durch einen nicht ausreichend schnellen metabolischen Abbau des Chelatkomplexes in der Leber zur Folge haben, wobei der systemische Effekt unerwünschte Nebenwirkungen mit sich bringen kann. Dieses Problem tritt insbesondere dann auf, wenn als Plasmaseparator ein Filter verwendet wird. Denn die geringe spezifische Trenneffizienz der Plasmafiltration führt dazu, dass ein großes Volumen an Vollblut den Filter durchlaufen muss, um eine bestimmte Menge an Plasma abtrennen zu können.

Vor diesem Hintergrund betrifft die Erfindung ein Disposable für die extrakorporale Blutbehandlung gemäß Anspruch 1, welches den gezielten Einsatz von Citrat mit Blick auf beide der oben beschriebenen Wirkungsweisen ermöglicht. Dies erfordert, zwei Schnittstellen für die Citratzugabe am Disposable vorzusehen, eine erste Schnittstelle für eine Zugabe in das Vollblut möglichst nahe am Zugangsport und andererseits eine zweite Schnittstelle für eine Zugabe in das Plasma stromabwärts des Separators bzw. der Plasmapumpe (Vermeidung eines retrograden Flusses).

Das erfindungsgemäße Disposable für die extrakorporale Blutbehandlung umfasst eine Blutzugangsleitung, einen Separator, eine Blutrückgabeleitung und eine Plasmaleitung. Es umfasst ferner wenigstens zwei Schnittstellen für die Zugabe von Citrat, wobei eine erste Schnittstelle in der Zugangsleitung und eine zweite Schnittstelle in der Plasmaleitung angeordnet ist.

Bei dem Disposable handelt es sich vorzugsweise um ein Apherese-Set, d.h. ein geschlossenes, steriles und nur einmal verwendbares Schlauchsystem einer Apherese- oder Plasmaseparationsvorrichtung.

In einer Ausführungsform umfasst das Disposable ferner wenigstens einen Abschnitt einer Citratleitung zur Verbindung mit der ersten und/oder zweiten Schnittstelle ("Komplettdisposable"). Die Citratleitung kann bereits an die erste oder zweite Schnittstelle gekoppelt sein, kann aber vorzugsweise noch ungekoppelt sein. Beispielsweise ist denkbar, dass die Citratleitung nicht an die erste oder zweite Schnittstelle gekoppelt ist sondern über einen Teil ihrer Länge oder über ihre vollständige Länge lösbar mit der Zugangsleitung oder der Plasmaleitung in Verbindung steht, beispielsweise oberflächlich entlang wenigstens eines Teilabschnitts mit dieser verschweißt oder verklebt ist. Im Falle einer Zugabe on Citrat könnte diese Verbindung dann durch Abziehen gelöst werden. Dies hat den Vorteil, dass vermieden wird, das Disposable durch separate, lose hängende Leitungen noch unübersichtlicher zu machen. In einer Ausführungsform weist die Vorrichtung an der Citratleitung eine integrierte Klemme oder ein integriertes Ventil auf. In einer Ausführungsform weist die Citratleitung zur Koppelung mit einer Schnittstelle Elemente eines mechanischen Verbindungssystems auf. Diese Verbindungselemente umfassen vorzugsweise einen männlichen Kegel eines Luer-Locks oder Luer-Ansatzes.

Alternativ ist denkbar, an dem Disposable keine Citratleitung vorzusehen.

Erfindungsgemäß weist das Disposable in der Zugangsleitung stromabwärts der ersten Schnittstelle und stromaufwärts des Separators eine Blutpumpe auf.

In einer Ausführungform weist das Disposable in der Plasmaleitung stromabwärts des Separators und stromaufwärts der zweiten Schnittstelle Elemente einer Plasmapumpe auf.

Die Blut- oder Plasmapumpe kann vollständig im Disposable integriert sein, allerdings bietet es sich aus Kostengünden an, die Pumpe maschinenseitig anzuordnen und nur gewisse Elemente der Pumpe am Disposable anzuordnen, die mit weiteren und maschinenseitig angeordneten Elementen der Pumpe wechselwirken und gemeinsam eine Pumpe bilden. Ein Beispiel ist eine peristaltische Pumpe, wobei am Disposable ein elastisch verformbares Schlauchsegment angeordnet sein kann, in welches maschinenseitig angeordnete Aktoren einer Pumpe eingreifen.

Erfindungsgemäß weist das Disposable in der Zugangsleitung stromaufwärts der ersten Schnittstelle ein Zugangsventil oder eine Zugangsklemme auf.

In einer Ausführungform weist das Disposable in der Plasmaleitung stromabwärts der zweiten Schnittstelle ein Plasmaventil oder Elemente einer Plasmaklemme auf.

Das Zugangs- oder Plasmaventil kann vollständig im Disposable angeordnet sein. Es handelt sich vorzugsweise um ein Ventil, das den Fluss im Disposable in einer Stellung vollständig unterbricht und in einer weiteren Stellung nicht behindert. Das Ventil kann so ausgebildet sein, dass es durch einen maschinenseitigen Aktor betätigt werden kann. Die Zugangs- oder Plasmaklemme kann vollständig im Disposable integriert sein, allerdings können auch nur gewisse Elemente der Klemme am Disposable angeordnet sein, die mit weiteren und maschinenseitig angeordneten Elementen der Klemme wechselwirken und gemeinsam eine Klemme bilden. Ein Beispiel ist ein elastisch verformbares (Schlauch-)segment am Disposable im Zusammenwirken mit einem maschinenseitig angeordneten Aktor.

Erfindungsgemäß weist das Disposable in der Zugangsleitung zwischen Z der Blutpumpe und der Zugangsklemme bzw. dem Zugangsventil einen Zugangsdrucksensor auf.

In einer Ausführungform weist das Disposable in der Plasmaleitung zwischen den Plasmapumpenelementen und den Plasmaklemmenelementen bzw. dem Plasmaventil einen Behandlungsdrucksensor oder Elemente eines Behandlungsdrucksensors auf.

Der Sensor kann vollständig im Disposable integriert sein, allerdings können aus Kostengründen auch nur gewisse Elemente des Sensors am Disposable angeordnet sein, die mit weiteren und maschinenseitig angeordneten Elementen des Sensors wechselwirken und gemeinsam einen Drucksensor bilden. Ein Beispiel ist eine elastische Wandung bzw. Membran am Disposable im Zusammenwirken mit einem maschinenseitig angeordneten Fühler.

In einer Ausführungform weist die erste Schnittstelle und/oder die zweite Schnittstelle Elemente eines mechanischen Verbindungssystems auf. Diese Verbindungselemente umfassen vorzugsweise einen weiblichen Kegel eines Luer-Locks oder Luer-Ansatzes. Dadurch wird das Disposable vielseitig einsetzbar, da ein männlicher Luer-Kegel an vielen maschinenseitigen Anschlüssen von bestehenden Geräten zu finden ist.

In einer Ausführungsform wird die erste und/oder zweite Schnittstelle durch die Einmündung einer zum Disposable gehörenden Citratleitung gebildet. Das mündungsabgewandte Ende der Citratleitung kann in diesem Fall Elemente eines mechanischen Verbindungssystems (wie eben beschrieben) aufweisen.

In einer Ausführungsform weist das Disposable an der ersten und/oder zweiten Schnittstelle eine integrierte Klemme oder ein integriertes Ventil auf. So kann eine nicht in Verwendung befindliche Schnittstelle vorzugsweise reversibel geschlossen werden.

In einer Ausführungform sind die erste Schnittstelle und die zweite Schnittstelle identisch ausgebildet. Dies ermöglicht eine Kopplung derselben Citratleitung an beide Schnittstellen.

In einer Ausführungform umfasst das Disposable in der Plasmaleitung stromabwärts der zweiten Schnittstelle eine Plasmabehandlungseinrichtung. Dabei kann es sich beispielsweise um partikuläres Sorptionsmittel oder eine poröse Sorptionsmatrix mit einer aktiven Oberfläche zur Adsorption von Blutkomponenten handeln. Ferner ist der Einsatz eines Filters, beispielsweise eines Hohlfaserfilters möglich.

In einer Ausführungsform handelt es sich bei dem Separator um einen Filter, vorzugsweise einen Plasmafilter. Dieser Filter kann als Hohlmembranfilter ausgestaltet sein.

Die Erfindung betrifft ferner eine Vorrichtung nach Anspruch 8 zur Durchführung einer Blutbehandlung, vorzugsweise einer Apherese. Die Vorrichtung weist ein erfindungsgemäßes Disposable auf. Ferner weist die Vorrichtung wenigstens eine Citratquelle auf, die mit der ersten Schnittstelle und/oder der zweiten Schnittstelle des erfindungsgemäßen Disposables gekoppelt ist. Die Koppelung erfolgt anhand einer (maschinen- oder disposableseitig angeordneten) Citratleitung. Gegebenenfalls ist die Vorrichtung so ausgebildet, dass die Citratquelle wahlweise sowohl mit der ersten als auch mit der zweiten Schnittstelle gekoppelt werden kann.

Sofern Citratquelle und Citratleitung maschinenseitig angeordnet sind, werden vorliegend unter dem Begriff Citratquelle die eigentliche Citratquelle und die Citratleitung zusammengefasst.

In einer Ausführungsform ist an oder stromaufwärts der Citratleitung eine Citratpumpe angeordnet. Anhand dieser Pumpe kann in der Citratleitung ein zur ersten und/oder zweiten Schnittstelle gerichteter Citratfluss erzeugt werden.

In einer Ausführungsform weist die Citratquelle zur Koppelung mit Citratleitung oder Schnittstelle Elemente eines mechanischen Verbindungssystems auf. Diese Verbindungselemente umfassen vorzugsweise einen männlichen Kegel eines Luer-Locks oder Luer-Ansatzes.
In einer Ausführungsform weist die Vorrichtung an der Citratleitung (zwischen Citratquelle und Schnittstelle) eine integrierte Klemme oder ein integriertes Ventil auf. Dies kann beispielsweise bei einem Anschlusswechsel erwünscht sein, oder dann, wenn während der Behandlung ausschließlich Heparin zur Koagulationshemmung verabreicht wird.

In einer Ausführungsform handelt es sich bei der Citratquelle um ein stromaufwärts der Citratleitung angeordnetes Reservoir, das mit Citrat befüllt ist.

In einer Ausführungsform weist die Vorrichtung eine Blutpumpe auf, die stromaufwärts des Separators und stromabwärts der ersten Schnittstelle in die Zugangsleitung des Disposables eingreift. In einer Ausführungsform weist die Vorrichtung eine Plasmapumpe auf, die stromabwärts des Separators und stromaufwärts der zweiten Schnittstelle in die Plasmaleitung des Disposables eingreift.

In einer Ausführungsform weist die Vorrichtung eine Zugangsklemme oder ein Zugangsventil auf, die oder das stromaufwärts der ersten Schnittstelle in die Zugangsleitung des Disposables eingreift. In einer Ausführungsform weist die Vorrichtung eine Plasmaklemme oder ein Plasmaventil auf, die oder das stromabwärts der zweiten Schnittstelle in die Plasmaleitung des Disposables eingreift. Es handelt sich vorzugsweise um ein Ventil oder eine Klemme, das oder die den Fluss im Disposable in einer Stellung vollständig unterbricht und in einer weiteren Stellung nicht behindert.

In einer Ausführungsform weist die Vorrichtung einen Zugangsdrucksensor auf, der derart ausgebildet ist, dass er den Flüssigkeitsdruck im Disposable zwischen der Blutpumpe und der Zugangsklemme bzw. dem Zugangsventil erfassen kann. In einer Ausführungsform weist die Vorrichtung einen Behandlungsdrucksensor auf, der derart ausgebildet ist, dass er den Flüssigkeitsdruck im Disposable zwischen der Plasmapumpe und der Plasmaklemme bzw. dem Plasmaventil erfassen kann.

Weiterhin beschäftigt sich die Erfindung mit der Frage, wie vor dem Hintergrund einer Verbindung einer Citratquelle mit einer Schnittstelle möglichst effizient und sicher ermittelt werden kann, ob eine ordnungsgemäße Verbindung hergestellt wurde. Die hier vorgestellte Lösung betrifft im Speziellen eine erfindungsgemäße Vorrichtung, in der durch die Möglichkeit der variablen Ankopplung einer Citratquelle an mehrere Schnittstellen eine Fehlerquelle entsteht. Die hier vorgestellte Lösung betrifft aber auch Vorrichtungen zur Blutbehandlung mit einer Citratquelle und einem Disposable im Allgemeinen.

Im Stand der Technik ist es grundsätzlich für medizinische Geräte bekannt, auf der Grundlage einer Druckmessung auf die Integrität von Leitungsverbindungen zu schließen.

So offenbart die EP 0 925 796 B1 ein Verfahren zur Erkennung eines Lecks in Flüssigkeitswegen einer Dialysevorrichtung, wobei der zu testende Abschnitt durch Klemmen abgekoppelt wird, im so erhaltenen isolierten System mit Hilfe der Blutpumpe ein Überdruck aufgebaut wird, und der Druckabfall gemessen und ausgewertet wird.

Die EP 1 031 358 B1 offenbart ein Kontrollverfahren für die Förderleistung von Flüssigkeit durch eine Leitung eines medizinischen Gerätes, wobei die Leitung stromabwärts einer Pumpe abgeklemmt wird und der Druckverlauf im abgeklemmten Bereich bei Maximalleistung der Pumpe überwacht wird.

Die EP 1 450 880 B1 offenbart eine Dialysevorrichtung, in der die Druckwellenamplitude im extrakorporalen Blutkreislauf detektiert und mit Blick auf eine mögliche Störung der Substituatzufuhr ausgewertet wird.

Die DE 10 2007 024 463 A1 offenbart ein Verfahren zur Überprüfung, ob eine Heparinpumpe korrekt an einen extrakorporalen Blutkreislauf angeschlossen wurde. Das Verfahren umfasst das Abklemmen eines Bereichs des extrakorporalen Blutkreislaufs, in dem sich die Blutpumpe, die Heparinleitung und ein Drucksensor befinden. Nach dem Abklemmen werden die Blutpumpe und die Heparinpumpe im Rahmen einer bestimmten Routine angesteuert, und der Druckverlauf wird gemessen und mit Blick auf eine mögliche Störung der Heparinzufuhr ausgewertet.

Die EP0111696 A2 offenbart eine Vorrichtung für extrakorporale Blutbehandlung mit einer Blutzugangsleitung, einem Separator, einer Blutrückgabeleitung, einer Plasmaleitung und zwei Schnittstellen für die Zugabe von Substanzen mit antikoagulativer Wirkung.

Um eine ordnungsgemäße Ankoppelung einer disposableseitigen Schnittstelle an einer Citratquelle sicherzustellen, sieht die Offenbarung ein Verfahren vor, welches Drucktests am Disposable umfasst.

Das offenbarte, nicht beanspruchte Verfahren zur Überprüfung der korrekten Ankopplung einer Citratquelle an eine flüssigkeitsführende Leitung einer Vorrichtung zur Durchführung einer Blutbehandlung sieht vor, dass anhand einer in der Citratquelle angeordneten Pumpe im Bereich der Ankopplung ein Überdruck erzeugt wird, der Anstieg und/oder anschließende Abfall dieses Drucks erfasst wird, und auf Grundlage des erfassten Druckanstiegs oder Druckabfalls unter Vorhaltung einer in der Vorrichtung hinterlegten Routine eine Aussage über die korrekte Ankopplung der Citratquelle getroffen wird. In einer Ausführungsform ist die Citratquelle maschinenseitig angeordnet und der Flüssigkeitskreislauf Teil eines Disposables. Unter der Ankopplung der Citratquelle wird insbesondere die Ankopplung einer zwischen Citratquelle und flüssigkeitsführenden Leitung befindlichen Citratleitung an die flüssigkeitsführende Leitung verstanden.

In einer Ausführungsform geht der Erzeugung eines Überdrucks ein Priming des Disposables voraus, gegebenenfalls unmittelbar voraus. Beispielsweise kann das Verfahren aber auch nach Ablauf einer gewissen Behandlungszeit und einem Anschlusswechsel für die Citratgabe erfolgen.

In einer Ausführungsform dient das Verfahren der Überprüfung der korrekten Ankopplung der Citratquelle an die erste oder die zweite Schnittstelle des Disposables der erfindungsgemäßen Vorrichtung. In dieser Ausführungsform wird der Überdruck im Bereich der ersten oder zweiten Schnittstelle anhand der Citratpumpe erzeugt.

In einer Ausführungsform dient das Verfahren zur Überprüfung der korrekten Ankopplung der Citratquelle an die erste Schnittstelle des Disposables der erfindungsgemäßen Vorrichtung. Dabei kann vorgesehen sein, dass der Überdruck im Bereich zwischen der Blutpumpe und der Zugangsklemme bzw. dem Zugangsventil erzeugt wird. Weiterhin kann vorgesehen sein, dass der Anstieg und/oder anschließende Abfall des Drucks anhand des Zugangsdrucksensors erfasst wird.

In einer Ausführungsform dient das Verfahren zur Überprüfung der korrekten Ankopplung der Citratquelle an die zweite Schnittstelle des Disposables der erfindungsgemäßen Vorrichtung. Dabei kann vorgesehen sein, dass der Überdruck im Bereich zwischen der Plasmapumpe und der Plasmaklemme bzw. dem Plasmaventil erzeugt wird. Weiterhin kann vorgesehen sein, dass der Anstieg und/oder anschließende Abfall des Drucks anhand des Behandlungsdrucksensors erfasst wird.

In einer Ausführungsform steht während des Verfahrens die Blutpumpe und/oder die Plasmapumpe still.

Weitere Einzelheiten und Vorteile ergeben sich aus den nachfolgend anhand der Figuren beschriebenen Ausführungsbeispielen. In den Figuren zeigen:
- Figur 1:: eine erfindungsgemäße Apheresevorrichtung, wobei die Citratquelle an die erste Schnittstelle gekoppelt ist; und
- Figur 2:: eine erfindungsgemäße Apheresevorrichtung, wobei die Citratquelle an die zweite Schnittstelle gekoppelt ist.

Das in den Figuren dargestellte Schaltbild zeigt die Vorrichtung 1 während des Primingvorgangs.

Die Vorrichtung 1 weist einen extrakorporalen Blutkreislauf 2 mit einer Zugangsleitung 3 von einem Patienten zu einem Plasmafilter 4 und einer Rückgabeleitung 5 vom Plasmafilter 4 zum Patienten auf. Das Vollblut wird mittels einer Blutpumpe 6 durch den extrakorporalen Blutkreislauf 2 gefördert. Durch den Plasmafilter 4 wird ein Teil des Blutplasmas abfiltriert und einem Plasmakreislauf 7 zugeführt. Das abgetrennte Plasma wird mittels einer Plasmapumpe 8 durch den Plasmakreislauf 7 gefördert. Stromabwärts der Plasmapumpe 8 kann sich je nach Art der Apheresevorrichtung im mit dem Bezugszeichen 9 markierten Bereich eine Behandlungseinrichtung befinden.

Vorzugsweise erstreckt sich der Bereich 9, in dem beispielsweise eine Adsorptionsvorrichtung bzw. Behandlungsvorrichtung angeordnet sein kann, zwischen den Klemmen 33 und 34.

Bei der Blutpumpe 6 und/oder der Plasmapumpe 8 kann es sich um eine peristaltische Pumpe handeln, wobei Schläuche oder andere flexible Abschnitte des Disposables als Pumpsegment dienen können, das in eine maschinenseitig angeordnete Pumpe eingespannt werden kann.

Der extrakorporale Blutkreislauf 2, der Plasmakreislauf 7 und der Plasmafilter 4 sind in der dargestellten Ausführungsform Teil des erfindungsgemäßen Disposables.

Das Disposable umfasst im gezeigten Ausführungsbeispiel noch weitere optionale Komponenten bzw. wechselwirkt noch mit weiteren optionalen, maschinenseitig angeordneten Komponenten. So ist eine Heparinpumpe 10 zwischen Blutpumpe 6 und Plasmafilter 4 an die Zugangsleitung 3 angeschlossen. Die Rückgabeleitung 5 umfasst eine Tropfkammer 11 mit einem Füllstandsdetektor 12. Weiter stromabwärts sind maschinenseitig ein optischer sowie ein Blasendetektor 13 an die Rückgabeleitung 5 gekoppelt. Am oder im Bereich des Rückgabeports 14 ist ein Reservoir 15 für eine Ca²⁺-ionenhaltige Lösung an die Rückgabeleitung 5 angeschlossen. Die zwischen dem Calciumreservoir 15 und der Rückgabeleitung 5 befindliche Calciumleitung 16 umfasst eine Tropfkammer 17 mit Füllstandsdetektor 18 und ist stromabwärts der Tropfkammer 17 an eine Calciumpumpe 19 gekoppelt. Der Plasmakreislauf umfasst einen Detektor 20 zur Erkennung von Blutzellen und Hämolyseprodukten.

Der Plasmakreislauf 7 kann je nach Einsatzweck der Apheresevorrichtung 1 derart realisiert sein, dass Plasma am stromabwärtigen Ende 21 des Plasmakreislaufs 7 der Rückgabeleitung 5 zugeführt wird. Ferner ist denkbar, dass das abgetrennte Plasma gesammelt wird.

Im Rahmen des dargestellten Schaltbildes (Primingvorgang) ist der Zugangsport 22 des Disposables an ein Reservoir 23 für eine Priminglösung gekoppelt. Bei der Priminglösung kann es sich beispielsweise um Kochsalzlösung handeln. Der Rückgabeport 14 des Disposables ist an einen Sammelbehälter 24 für verbrauchte Primingflüssigkeit gekoppelt. Das stromabwärtige Ende 21 des Plasmakreislaufs 7 des Disposables ist ebenfalls an einen Sammelbehälter 25 für verbrauchte Primingflüssigkeit gekoppelt.

Drucksensoren sind an mehreren Stellen des Disposables vorhanden bzw. angekoppelt, um den jeweiligen Flüssigkeitsdruck zu messen. So befindet sich zwischen Zugangsport 22 und Blutpumpe 6 ein Zugangsdrucksensor 26. Zwischen Blutpumpe 6 und Plasmafilter 4 ist ein Filterdruckmesser 27 angeordnet. Stromabwärts des Plasmafilters 4 ist in oder an der Rückgabeleitung 5 ein Rücklaufdruckmesser 28 angeordnet. In oder an der Plasmaleitung 7 ist stromabwärts des Plasmafilters 4 und stromaufwärts der Plasmapumpe 8 ein Plasmadruckmesser 29 angeordnet. Zwischen der Plasmapumpe 8 und dem stromabwärtigen Ende 21 des Plasmakreislaufs 7 ist ein Behandlungesdrucksensor 30 angeordnet.

Ferner sind an mehreren Stellen des Disposables Klemmen vorhanden, um den Fluss unterbrechen oder drosseln zu können. Alternativ zu Klemmsegmenten können auch Rückschlagventile zum Einsatz kommen. Stromaufwärts der Blutpumpe 6 und des Zugangsdrucksensors 26 befindet sich eine Zugangsklemme 31. In oder an der Rückgabeleitung 5 ist eine Rücklaufklemme 32 angeordnet, die sich im gezeigten Ausführungsbeispiel stromabwärts von Tropfkammer 11 und Blasendetektor 13 sowie stromaufwärts von der Ankoppelungsstelle der Calciumleitung 16 befindet. In oder an der Plasmaleitung 7 ist stromabwärts der Plasmapumpe 8 und des Behandlungsdrucksensors 30 eine Plasmaklemme 33 angeordnet. Denkbar ist auch, stromabwärts der Plasmapumpe 8 und des Behandlungsdrucksensors 30 zwei Plasmaklemmen 33 und 34 vorzusehen.

Das Disposable umfasst weiterhin zwei Schnittstellen 35 und 36 zur Ankoppelung einer Citratleitung 37. Beide Schnittstellen 35 und 36 umfassen einen Zugang und erlauben die Zugabe von Citrat in die Leitungen des Disposables. Die Citratleitung ist ebenfalls Teil des Disposables, ist im Lieferzustand jedoch nicht an die Schnittstellen angekoppelt. Vielmehr ist diese an eine bestehende Leitung des Disposables, beispielsweise die Zugangsleitung derart angeschweißt, dass diese angenommen und angesteckt werden kann.

Die erste Schnittstelle 35 ist am oder im Bereich des Zugangsports 22 angeordnet. Sie befindet sich stromaufwärts der Blutpumpe 6 und des Zugangsdrucksensors 26. Denkbar ist, dass die erste Schnittstelle 35 weiterhin stromabwärts der Zugangsklemme 31 angeordnet ist.

Die zweite Schnittstelle 36 ist stromabwärts der Plasmapumpe 8 und stromaufwärts der Plasmaklemme 33 an der Plasmaleitung 7 angeordnet. Denkbar ist, dass die zweite Schnittstelle 36 weiterhin stromaufwärts einer im mit dem Bezugszeichen 9 markierten Bereich befindlichen Behandlungseinrichtung angeordnet ist.

Vorzugsweise erstreckt sich der Bereich 9, in dem beispielsweise eine Adsorptionsvorrichtung bzw. Behandlungsvorrichtung angeordnet sein kann, zwischen den Klemmen 33 und 34, wie dies aus den Figuren ersichtlich ist.

Beide Schnittstellen 35 und 36 sind vorzugsweise identisch ausgebildet. Vorzugsweise umfassen beide Schnittstellen 35 und 36 einen weiblichen Konus eines Luer-Konnektors. Beide Schnittstellen 35 und 36 umfassen in einer Ausführungsform eine Vorrichtung zum Verschließen des Zugangs, beispielsweise eine Klemme oder ein Ventil.

An der zu den disposableseitigen Schnittstellen 35 und 36 korrespondierenden Schnittstelle 38 der Citratleitung 37 ist vorzugsweise ein männlicher Konus eines Luer-Konnektors vorhanden. Auch die Schnittstelle 38 kann eine Vorrichtung zum Verschließen des Zugangs umfassen, beispielsweise eine Klemme oder ein Ventil.

In der Citratleitung 37 ist eine Citratpumpe 39 angeordnet, die das Citrat zur Schnittstelle 38, und, sofern diese Schnittstelle an eine Schnittstelle 35 oder 36 angekoppelt ist, in die Leitungen des extrakorporalen Blut- bzw Plasmakreislaufs fördern kann. Bei der Citratpumpe 39 kann es sich beispielsweise um eine peristaltische Pumpe handeln. Ferner ist im gezeigten Ausführungsbeispiel in der Citratleitung 37 stromaufwärts der Citratpumpe 39 eine Tropfkammer 40 mit Tropfenzähler und/oder Füllstandsdetektor angeordnet. Die Citratleitung bezieht das Citrat aus einer Citratquelle 40, die im gezeigten Ausführungsbeispiel als maschinenseitig angeordnetes Reservoir ausgebildet ist.

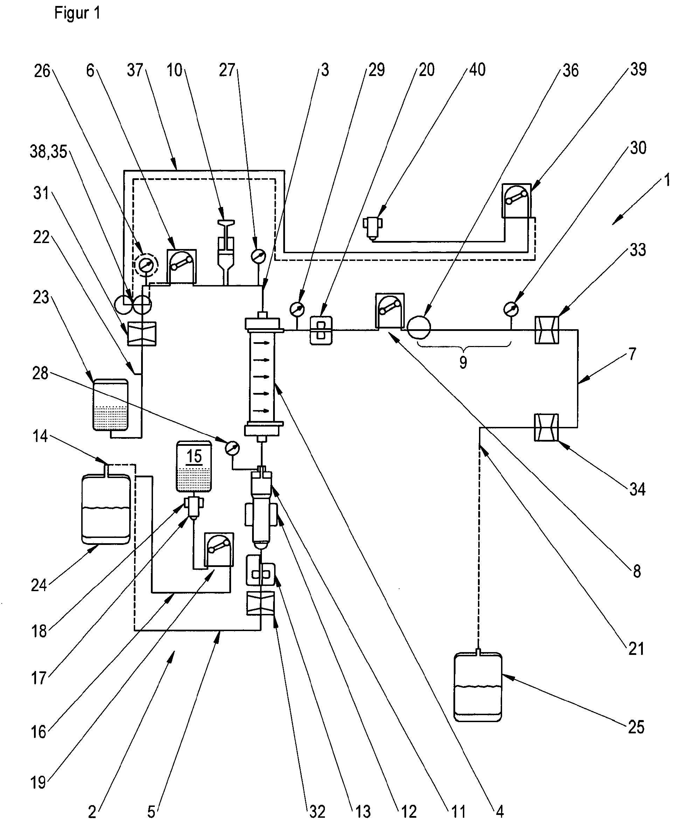

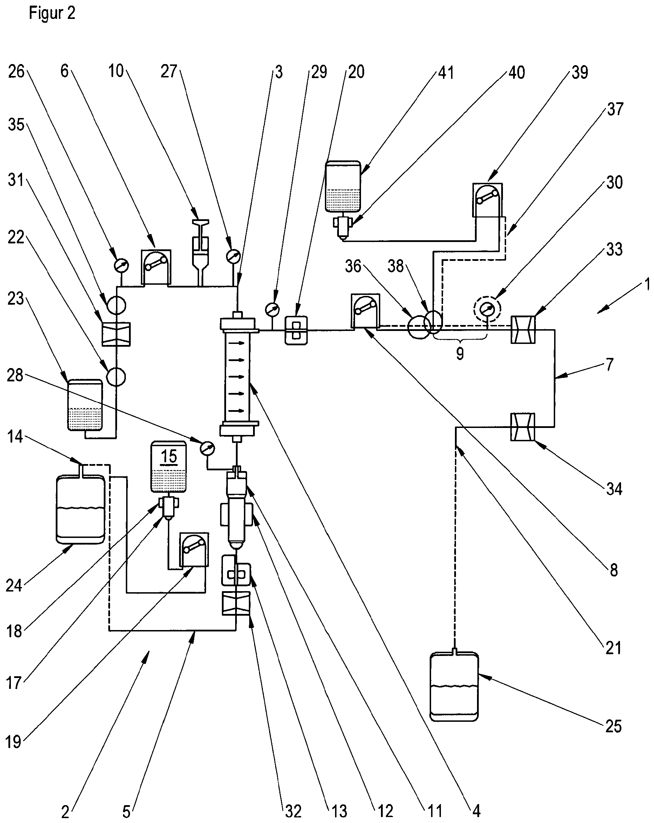

Citrat kann, wie eingangs dargestellt, sowohl zur Antikoagulation von Blut als auch zur Abreicherung von Ca²⁺-Ionen im Plasma eingesetzt werden. Die Zugabe erfolgt je nach Verwendungszweck vorzugsweise an unterschiedlichen Stellen des extrakorporalen Kreislaufs. Die Erfindung schlägt die Kombination zweier für den jeweiligen Zweck angepassten Disposablevarianten zu einem einzigen Disposable vor, das idealerweise einen Wechsel zwischen unterschiedlichen Zugabestellen auch während der Behandlung ermöglicht. Dies ist auch im in den Figuren 1 und 2 dargestellten Disposable der Fall. Der Wechsel ist für den Anwender komfortabel durchzuführen und dennoch sicher (Überprüfung durch Druckmessung).

In Figur 1 ist ein Schaltbild dargestellt, wobei die Citratleitung 37 an die erste Schnittstelle 35 des Disposables gekoppelt ist. Die erste Schnittstelle 35 ist so angeordnet, dass die Zugabe des Citrats ins Vollblut unmittelbar nach Entnahme aus dem Körper erfolgen kann. Der Anschluss der Citratleitung 37 an die erste Schnittstelle 35 erfolgt dann, wenn das Citrat während einer Behandlung zum Zwecke der Koagulationshemmung zugegeben werden soll.

In Figur 2 ist ein Schaltbild dargestellt, wobei die Citratleitung 37 an die zweite Schnittstelle 36 des Disposables gekoppelt ist. Die zweite Schnittstelle 36 ist so angeordnet, dass die Zugabe des Citrats ins Plasma stromabwärts der Plasmapumpe 8 und stromaufwärts einer etwaigen Behandlungseinrichtung befindet. Der Anschluss der Citratleitung 37 an die zweite Schnittstelle 36 erfolgt dann, wenn das Citrat während einer Behandlung zum Zwecke der Immunsuppression zugegeben werden soll.

Ein Umrüsten bestehender Blutbehandlungsgeräte zur Anpassung an das neue Disposable ist in den meisten Geräten nicht erforderlich. Ferner ist eine Unterbrechung der Behandlung zum Wechsel des Anschlusses der Citratleitung 37 zwischen erster Schnittstelle 35 und zweiter Schnittstelle 36 in vielen Fällen nicht erforderlich.

Im Rahmen einer Ausführungsform des erfindungsgemäßen Verfahrens kann anhand einer Druckmessung überprüft werden, ob die Citratleitung 37 ordnungsgemäß an der ersten Schnittstelle 35 angeschlossen wurde. Diese Ausführungsform des Verfahrens lässt sich mit Blick auf Figur 1 erklären. Beispielsweise werden zunächst die Citratleitung 37 an die erste Schnittstelle 35 angekoppelt und ein Primingvorgang für das Disposable und die Citratleitung durchgeführt. Anschließend erfolgt eine Stillegung der Blutpumpe 6. Die Zugangsleitung 3 ist durch die stillstehende Blutpumpe 6 stromabwärts der ersten Schnittstelle 35 unterbrochen. Stromaufwärts der ersten Schnittstelle wird die Zugangsleitung 3 ebenfalls unterbrochen, beispielsweise durch Schließen der Zugangsklemme 31 oder eines Ventils am Zugangsport 22. In einem weiteren Schritt wird die Citratpumpe 39 betrieben, sodass sich in der Citratleitung 37, an der ersten Schnittstelle 35 sowie im abgeklemmten Bereich der Zugangsleitung 3 ein Überdruck aufbaut. Der Druck kann anhand des im bzw. am abgeklemmten Bereich befindlichen Zugangsdrucksensors 26 erfasst werden.

Im Rahmen einer weiteren Ausführungsform des offenbarten, nicht beanspruchten Verfahrens kann anhand einer Druckmessung überprüft werden, ob die Citratleitung 37 ordnungsgemäß an der zweiten Schnittstelle 36 angeschlossen wurde. Diese Ausführungsform des Verfahrens lässt sich mit Blick auf Figur 2 erklären. Beispielsweise wird zunächst die Citratleitung 37 an die zweite Schnittstelle 36 angekoppelt. Anschließend erfolgt eine Stillegung der Plasmapumpe 8 und die Unterbrechung der Plasmaleitung 7 durch Schließen der Plasmaklemme 33. Anschließend wird die Citratpumpe 39 betrieben, sodass sich in der Citratleitung 37, an der zweiten Schnittstelle 36 sowie im abgeklemmten Bereich der Plasmaleitung 7 ein Überdruck aufbaut. Der Druck kann anhand des im bzw. am abgeklemmten Bereich befindlichen Behandlungsdrucksensors 30 erfasst werden.

In beiden Fällen ist der Bereich des Druckaufbaus in den Figuren mit einer gestrichelten Linie markiert.

In beiden Fällen wird bei Erreichen eines bestimmten Volumens oder nach Ablauf einer bestimmten (auch ggf. vom Benutzer frei wählbaren) Zeit die Citratpumpe 39 abgeschaltet. Anschließend wird der Druckverlauf im abgeklemmten Bereich erfasst. Der Verlauf des Druckanstiegs bei Betrieb der Citratpumpe 39 und/oder der Verlauf des Druckabfalls nach Abschalten der Citratpumpe können dazu verwendet werden, um etwaige Fehler bei der Konnektierung der Citratleitung 37 an die jeweilige Schnittstelle 35 oder 36 zu erfassen. Dieser Test dient in einer Ausführungsform vornehmlich zur Sicherstellung der richtigen Anschlussposition der Citratleitung: es wird überprüft, ob die Wahl des Citratzugabemodus und die dazu gehörige Verbindung konsistent sind. Da sich die Zugaberaten für beide Anschlusspositionen deutlich unterscheiden können, wäre bei einer Fehlkonnektion das Risiko einer Fehldosierung gegeben. Ein weiterer möglicher zu detektierender Fehler umfasst Undichtigkeiten im Verbindungsbereich oder eine fehlende Zugabe von Citrat in die jeweilige Schnittstelle des Disposables. Nach Beendigung des Verfahrens werden die Pumpen 6 bzw. 8 wieder in Betrieb genommen und/oder etwaige Klemmen 31 bzw. 33 werden gelöst, um im vormals abgeklemmten Bereich wieder Systemdruck herzustellen und einen Flüssigkeitsdurchlauf zu gewährleisten.

So kann durch Druckmodulation überprüft werden, ob eine Citratleitung korrekt am Disposable angeschlossen wurde.

## Patentansprüche

1. Disposable für die extrakorporale Blutbehandlung, vorzugsweise Apherese-Set, das eine Blutzugangsleitung (3), einen Separator (4), eine Blutrückgabeleitung (5), eine Plasmaleitung (7) und zwei Schnittstellen (35, 36) für die Zugabe von Citrat umfasst, wobei
eine erste Schnittstelle (35) in der Blutzugangsleitung (3) und eine zweite Schnittstelle (36) in der Plasmaleitung (7) angeordnet ist,
wobei das Disposable in der Blutzugangsleitung (3) stromabwärts der ersten Schnittstelle (35) und stromaufwärts des Separators (4) eine Blutpumpe (6) aufweist, das Disposable in der Blutzugangsleitung (3) stromaufwärts der ersten Schnittstelle (35) ein Zugangsventil (31) oder eine Zugangsklemme (31) aufweist, und das Disposable in der Blutzugangsleitung (3) zwischen der Blutpumpe (6) und dem Zugangsventil (31) oder der Zugangsklemme (31) einen Zugangsdrucksensor (26) aufweist.

2. Disposable nach Anspruch 1, **dadurch gekennzeichnet, dass** das Disposable ferner eine Citratleitung zur Verbindung mit der ersten und/oder zweiten Schnittstelle umfasst, wobei die Citratleitung vorzugsweise nicht an die erste oder zweite Schnittstelle gekoppelt ist und lösbar an einem weiteren Teil des Disposables anhaftet.

3. Disposable nach einem der vorhergehenden Ansprüche, **dadurch gekennzeichnet, dass** das Disposable in der Plasmaleitung (7) stromabwärts der zweiten Schnittstelle (36) ein Plasmaventil (33) oder eine Plasmaklemme (33) umfasst.

4. Disposable nach Anspruch 3, **dadurch gekennzeichnet, dass** das Disposable in der Plasmaleitung (7) stromabwärts des Separators (4) und stromaufwärts der zweiten Schnittstelle (36) einer Plasmapumpe (8) aufweist, wobei das Disposable in der Plasmaleitung (7) zwischen der Plasmapumpe und der Plasmaklemme bzw. dem Plasmaventil einen Behandlungsdrucksensor (30) umfasst.

5. Disposable nach einem der vorhergehenden Ansprüche, **dadurch gekennzeichnet, dass** die erste Schnittstelle (35) und/oder zweite Schnittstelle (36) ein mechanisches Verbindungssystem aufweist, vorzugsweise einen weiblichen Kegel eines Luer-Locks oder Luer-Ansatzes.

6. Disposable nach einem der vorhergehenden Ansprüche, **dadurch gekennzeichnet, dass** die erste Schnittstelle (35) und/oder zweite Schnittstelle (36) eine integrierte Klemme oder ein integriertes Ventil aufweist.

7. Disposable nach einem der vorhergehenden Ansprüche, **dadurch gekennzeichnet, dass** die erste Schnittstelle (35) und die zweite Schnittstelle (36) identisch ausgebildet sind.

8. Vorrichtung zur Durchführung einer extrakorporalen Blutbehandlung,
wobei die Vorrichtung ein Disposable nach einem der vorhergehenden Ansprüche aufweist und die Vorrichtung eine Citratquelle (40) aufweist, die mit der ersten Schnittstelle (35) oder der zweiten Schnittstelle (36) gekoppelt ist.

9. Vorrichtung nach Anspruch 8, **dadurch gekennzeichnet, dass** die Citratquelle (40) zur Koppelung mit der Citratleitung (37) oder der ersten Schnittstelle (35) und/oder der zweiten Schnittstelle (36) ein mechanisches Verbindungssystem aufweist, vorzugsweise einen männlichen Kegel eines Luer-Locks oder Luer-Ansatzes.

10. Vorrichtung nach einem der Ansprüche 8 oder 9, **dadurch gekennzeichnet, dass** die Vorrichtung eine Plasmaklemme (33) oder ein Plasmaventil (33) umfasst, die oder das stromabwärts der zweiten Schnittstelle (36) in die Plasmaleitung (7) des Disposables eingreift.

11. Vorrichtung nach Anspruch 10, **dadurch gekennzeichnet, dass** die Vorrichtung eine Plasmapumpe (8) aufweist, die stromabwärts des Separators (4) und stromaufwärts der zweiten Schnittstelle (36) in die Plasmaleitung (7) des Disposables eingreift, wobei die Vorrichtung einen Adsorberdrucksensor (30) umfasst, der derart ausgebildet ist, dass er den Flüssigkeitsdruck im Disposable zwischen der Plasmapumpe (8) und der Plasmaklemme (33) bzw. dem Plasmaventil (33) erfassen kann.

## Claims

1. Disposable for extracorporeal blood treatment, preferably apheresis set, which comprises a blood admission line (3), a separator (4), a blood return line (5), a plasma line (7) and two interfaces (35, 36) for the addition of citrate, wherein a first interface (35) is arranged in the blood admission line (3) and a second interface (36) is arranged in the plasma line (7), wherein in the blood admission line (3) the disposable has a blood pump (6) downstream of the first interface (35) and upstream of the separator (4), in the blood admission line (3) the disposable has an access valve (31) or an access clamp (31) upstream of the first interface (35), and in the blood admission line (3) the disposable has an access pressure sensor (26) between the blood pump (6) and the access valve (31) or the access clamp (31).

2. Disposable according to claim 1, **characterised in that** the disposable further comprises a citrate line for connection to the first and/or second interface, wherein the citrate line is preferably not coupled to the first or second interface and adheres detachably to another part of the disposable.

3. Disposable according to one of the preceding claims, **characterised in that** the disposable comprises a plasma valve (33) or a plasma clamp (33) in the plasma line (7) downstream of the second interface (36).

4. Disposable according to claim 3, **characterised in that** in the plasma line (7) the disposable has a plasma pump (8) downstream of the separator (4) and upstream of the second interface (36), wherein the disposable comprises a treatment pressure sensor (30) in the plasma line (7) between the plasma pump and the plasma clamp or the plasma valve.

5. Disposable according to any one of the preceding claims, **characterised in that** the first interface (35) and/or second interface (36) has a mechanical connection system, preferably a female taper fitting of a Luer lock or Luer connector.

6. Disposable according to any one of the preceding claims, **characterised in that** the first interface (35) and/or second interface (36) has an integrated clamp or an integrated valve.

7. Disposable according to any one of the preceding claims, **characterised in that** the first interface (35) and the second interface (36) are designed identically.

8. Device for carrying out an extracorporeal blood treatment, wherein the device has a disposable according to one of the preceding claims and the device has a citrate source (40), which is coupled to the first interface (35) or the second interface (36).

9. Device according to claim 8, **characterised in that** the citrate source (40) for coupling to the citrate line (37) or the first interface (35) and/or the second interface (36) has a mechanical connection system, preferably a male taper fitting of a Luer lock or Luer connector.

10. Device according to one of claims 8 or 9, **characterised in that** the device comprises a plasma clamp (33) or a plasma valve (33), which clamp or valve engages in the plasma line (7) of the disposable downstream of the second interface (36).

11. Device according to claim 10, **characterised in that** the device has a plasma pump (8), which engages downstream of the separator (4) and upstream of the second interface (36) in the plasma line (7) of the disposable, wherein the device comprises an adsorber pressure sensor (30), which is designed so that it can detect the fluid pressure in the disposable between the plasma pump (8) and the plasma clamp (33) or the plasma valve (33).

## Revendications

1. Article à usage unique pour le traitement extracorporel du sang, de préférence kit d'aphérèse, qui comprend une ligne d'arrivée du sang (3), un séparateur (4), une ligne de retour du sang (5), une ligne de plasma (7) et deux interfaces (35, 36) pour l'ajout de citrate, dans lequel une première interface (35) est disposée dans la ligne d'arrivée du sang (3) et une seconde interface (36) dans la ligne de plasma (7), dans lequel l'article à usage unique comporte, dans la ligne d'arrivée du sang (3), en aval de la première interface (35) et en amont du séparateur (4), une pompe à sang (6), l'article à usage unique comporte, dans la ligne d'arrivée du sang (3), en amont de la première interface (35), une valve d'arrivée (31) ou une pince d'arrivée (31), et l'article à usage unique comporte, dans la ligne d'arrivée du sang (3), entre la pompe à sang (6) et la valve d'arrivée (31) ou la pince d'arrivée (31), un capteur de pression d'accès (26).

2. Article à usage unique selon la revendication 1, **caractérisé en ce qu'**il comprend en outre une ligne de citrate pour la liaison à la première et/ou la seconde interface, la ligne de citrate n'étant de préférence pas raccordée à la première ou seconde interface et adhérant de manière détachable à une autre partie de l'article à usage unique.

3. Article à usage unique selon l'une des revendications précédentes, **caractérisé en ce qu'**il comprend, dans la ligne de plasma (7), en aval de la seconde interface (36), une valve pour le plasma (33) ou une pince pour le plasma (33).

4. Article à usage unique selon la revendication 3, **caractérisé en ce qu'**il comporte, dans la ligne de plasma (7), en aval du séparateur (4) et en amont de la seconde interface (36), une pompe à plasma (8), l'article à usage unique comprenant, dans la ligne de plasma (7), entre la pompe à plasma et la pince pour le plasma ou la valve pour le plasma, un capteur de pression de traitement (30).

5. Article à usage unique selon l'une des revendications précédentes, **caractérisé en ce que** la première interface (35) et/ou la seconde interface (36) comporte un système de liaison mécanique, de préférence un cône femelle d'un Luer-Lock ou d'un embout Luer.

6. Article à usage unique selon l'une des revendications précédentes, **caractérisé en ce que** la première interface (35) et/ou la seconde interface (36) comporte une pince intégrée ou une valve intégrée.

7. Article à usage unique selon l'une des revendications précédentes, **caractérisé en ce que** la première interface (35) et la seconde interface (36) sont réalisées identiques.

8. Dispositif permettant d'exécuter un traitement extracorporel du sang,
le dispositif comportant un article à usage unique selon l'une des revendications précédentes et le dispositif comportant une source de citrate (40) qui est raccordée à la première interface (35) ou la seconde interface (36).

9. Dispositif selon la revendication 8, **caractérisé en ce que** la source de citrate (40) comporte, pour le raccordement à la ligne de citrate (37) ou à la première interface (35) et/ou à la seconde interface (36), un système de liaison mécanique, de préférence un cône mâle d'un Luer-Lock ou d'un embout Luer.

10. Dispositif selon l'une des revendications 8 ou 9, **caractérisé en ce qu'**il comprend une pince pour le plasma (33) ou une valve pour le plasma (33), qui s'engage dans la ligne de plasma (7) de l'article à usage unique en aval de la seconde interface (36).

11. Dispositif selon la revendication 10, **caractérisé en ce qu'**il comprend une pompe à plasma (8), qui s'engage dans la ligne de plasma (7) de l'article à usage unique, en aval du séparateur (4) et en amont de la seconde interface (36), le dispositif comprenant un capteur de pression d'adsorbant (30), qui est conçu de manière à pouvoir détecter la pression de liquide dans l'article à usage unique entre la pompe à plasma (8) et la pince pour le plasma (33) ou la valve pour le plasma (33).
